# EUROPÄISCHE PATENTSCHRIFT

(11) **EP 3 226 834 B1**
(45) Veröffentlichungstag und Bekanntmachung des Hinweises auf die Patenterteilung: **17.06.2020**
(21) Anmeldenummer: 15810619.5
(22) Anmeldetag: 23.11.2015
(51) Int. Cl.: A61K 8/96, A61K 8/37, A61K 8/39, A61K 8/44, A61K 8/46, A61K 8/81, A61K 8/87, A61Q 5/02, A61Q 19/10

(54) **REINIGUNGSZUBEREITUNGEN ENTHALTEND AKTIVKOHLE**
CLEANING PREPARATIONS CONTAINING ACTIVATED CARBON
PRÉPARATIONS DE NETTOYAGE CONTENANT DU CHARBON ACTIF

(30) Priorität: 05.12.2014 DE 102014225009
(43) Veröffentlichungstag der Anmeldung: 11.10.2017
(73) Patentinhaber: Beiersdorf AG, 20253 Hamburg (DE)
(72) Erfinder: DIPPE, Rixa, 20255 Hamburg (DE); SORS, Nathalie, 22307 Hamburg (DE); HACHMANN, Tobias, 20251 Hamburg (DE); NEMNICH, Julie, 22589 Hamburg (DE); WILKEN, Maren, 22848 Norderstedt (DE)
(86) Internationale Anmeldenummer: PCT/EP2015/077307
(87) Internationale Veröffentlichungsnummer: WO 2016/087241

(56) Entgegenhaltungen:
- EP-A1- 1 081 216
- JP-A- 2002 167 325
- US-B1- 6 210 694
- DATABASE GNPD [Online] MINTEL; März 2011 (2011-03), "Charcoal Foam Cleanser", XP002754012, Database accession no. 1505578
- DATABASE GNPD [Online] MINTEL; April 2008 (2008-04), "Deep Shower", XP002754013, Database accession no. 891893
- Übersetzung des Dokuments JP 2002 167325 ins Deutsche

## Beschreibung

Reinigungsprodukte sollen die Haut und die Haare reinigen. Nach dem Reinigungsprozess stellt sich beim Anwender im Idealfall ein Gefühl von Sauberkeit, Frische und Wohlbefinden ein. Der Mensch hat von je her das Bedürfnis gehabt sich zu reinigen, d.h. Schmutz von der Haut oder aus den Haaren zu entfernen. Über die Zeit haben sich die Reinigungsprozesse gewandelt wie auch die Produkte, die zur Reinigung verwendet werden. Heutzutage wird die Haut beispielsweise durch Waschen, Duschbäder oder Wannenbäder gereinigt, während die Haare oftmals während des Duschbades oder Wannenbades mit gereinigt werden oder auch separat gewaschen werden.

Körperreinigungsprodukte dienen in erster Linie dazu Schmutz von der Hautoberfläche zu entfernen. Der Schmutzfilm besteht aus festen oder flüssigen Komponenten, die von außen auf die Haut gelangt sind. Ebenfalls zum Schmutzfilm gehören überschüssige Hautlipide und abgestorbene Körperzellen. Durch oberflächenaktive Inhaltsstoffe in den Reinigungszusammensetzungen gelangen die Komponenten des Schmutzfilms in die Waschflotte und werden durch den Abspülprozess von der Haut entfernt.

Auch für die Reinigung der Haare sind Zubereitungen, die sich durch eine gute bis sehr gute Reinigungsleistung auszeichnen und zugleich eine angenehme Sensorik haben, beim Kunden sehr erwünscht. Darüber hinaus spielt das Aussehen einer Zubereitung eine wichtige Rolle für die Akzeptanz beim Verbraucher.

Das Haar besteht aus dem Haarschaft und der Haarwurzel. Der Haarschaft ist der keratinisierte, tote Teil eines Haares, der aus der Haut herausragt. Die Gesamtheit der Haarschäfte bildet das von außen sichtbare Haar. Der Haarschaft ist aus drei Schichten aufgebaut, einem zentralen Teil, dem sogenannten Haarmark (Medulla), der allerdings beim Menschen zurückgebildet ist und oft gänzlich fehlt, der Rinde (Cortex) und der äußeren, bis zu zehn Lagen starken Schuppenschicht (Cuticula), die das ganze Haar umhüllt. Die Haarwurzel ist in der Haut lokalisiert und stellt den lebenden Teil dar. In den Haarwurzeln erfolgt die ständige Neubildung des Haares.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Ein Ziel der Haarpflege ist es, dass der Naturzustand des frisch nachgewachsenen Haares über einen möglichst langen Zeitraum zu erhalten bleibt und im Falle eines Verlusts ihn wieder herzustellen. Seidiger Glanz, geringe Porosität und ein angenehmes, glattes Gefühl gelten als Merkmale für natürliches, gesundes Haar. Die Reinigung der Haare soll Verschmutzungen aus den Haaren entfernen. Dies können Verschmutzungen sein, die von außen auf die Haare gelangt sind, wie Staub, Ruß, Fett, Öl und andere oder Verschmutzungen, die ursprünglich von der Kopfhaut stammen, wie zum Beispiel Talg oder abgestorbenen Hautzellen, die sich in der Regel in Form kleiner Schüppchen ablösen. Größere, zusammenhängende Hautpartikel können als Schuppen sichtbar werden.

Es gibt jedoch Situationen, in denen eine intensivere Reinigung erforderlich ist. Dies ist zum Beispiel der Fall bei stark verschmutzten Hautoberflächen. Eine solch starke Verschmutzung ist bei manchen Berufsgruppen anzutreffen, z.B. bei Automechanikern. Ölhaltige Verschmutzungen lassen sich oftmals mit gewöhnlichen festen oder flüssigen Seifen oder Handreinigungsmitteln nicht entfernen.

Auch bei einer tiefenwirksamen Hautreinigung, bei der abgestorbene Hornzellen der oberen Hautschicht abgetragen werden sollen um ein gleichmäßigeres Hautbild zu erhalten, reichen normale Hautreinigungsmittel nicht aus.

Um eine tiefenwirksame gründliche Reinigung zu erzielen gibt es verschiedene Möglichkeiten.

Zum einen kann in den Reinigungszubereitungen der Anteil der Tenside, die eine sehr gute Reinigungsleistung erzielen, erhöht werden. Zu diesen Tensiden werden in der Regel anionische Tenside gerechnet, aber auch amphotere Tenside auf der Basis von Betain und auch einige nichtionische Tenside zeigen eine gute Reinigungswirkung. Bei der Erhöhung des Anteils an anionischen Tensiden in einer Zubereitung ist jedoch damit zu rechnen, dass eine Haut-reizende Wirkung der entsprechenden Zubereitung, insbesondere bei häufigem und intensivem Gebrauch, auftreten kann.

Eine andere Möglichkeit besteht darin, den Reinigungszubereitungen abrasive Partikel zu zusetzen. Diese Partikeln bewirken eine zusätzliche mechanische Reinigung. Dadurch gelingt es in vielen Fällen starke und festsitzende Verschmutzungen von der Haut zu entfernen und eine tiefenwirksame Reinigung zu erzielen. Als abrasive Partikel werden in vielen Fällen Polyethylenpartikel oder pulverisierte Pflanzenbestandteile wie beispielsweise Obstkerne oder Nussschalen eingesetzt. Derartige Partikel werfen jedoch Probleme auf. Die Polyethylenpartikel werden nur sehr schlecht bis gar nicht in den Klärwerken biologisch abgebaut. In der Folge akkumulieren sie in den Flüssen und Meeren. Sie stellen damit eine Gefahr für die Fischpopulationen dar.

Auch die Partikel aus pulverisierten Pflanzenbestandteilen weisen Nachteile auf, denn diese Partikel sind anfällig für Verkeimungen. Um diese Verkeimungen zu verhindern, werden die Partikel aus pulverisierten Pflanzenbestandteilen häufig mit Gamma-Bestrahlung behandelt, einem Verfahren, dem die Verbraucher jedoch kritisch gegenüber stehen.

Es bestand also Bedarf Reinigungszubereitungen zur Verfügung zu stellen, die geeignet sind, starke Verschmutzungen zu entfernen und eine tiefenwirksame Reinigung der Hautoberfläche zu bewirken ohne die oben beschriebenen Nachteile aufzuweisen.

Ebenso können Haare starke Verschmutzungen aufweisen, die insbesondere bei bestimmten Berufsgruppen auftreten können. Erwähnt seien hier Automechaniker, Landwirte und andere. Auch in diesen Fällen ist eine tiefenwirksame Reinigung erforderlich.

Zubereitungen, die diese Anforderungen erfüllen, dürfen darüber hinaus keine Defizite in Bezug auf die Milde und Pflegeleistung aufweisen. Dies bedeutet, dass Produkte für eine tiefenwirksame Reinigung eine wenigstens vergleichbare Milde wie bereits auf dem Markt befindliche Produkte aufweisen müssen und darüber hinaus eine Pflegewirkung zeigen, die ebenfalls derjenigen Pflegewirkung der bereits auf dem Markt befindlich Produkt nicht nachsteht.

Die Aufgabe, eine tiefenwirksame Reinigung zu erzielen, wird durch das Bereitstellen von Reinigungszubereitungen gelöst, die Aktivkohle enthalten.

Aktivkohle wird auch A-Kohle oder medizinische Kohle genannt. Es handelt sich um ein feinkörniges, poröses Material, das zu einem großen Teil aus Kohlenstoff besteht. Als Ausgangsstoffe können pflanzliche, tierische, mineralische oder petrochemische Materialen verwendet werden. Durch das Herstellungsverfahren, eine Gasaktivierung oder chemische Aktivierung entsteht die Aktivkohle, die sehr porös ist und eine sehr große innere Oberfläche aufweist. Diese beiden Merkmale bestimmen die Eigenschaften der Aktivkohle, sie wird in vielen Bereichen als Adsorptionsmittel verwendet. Aktivkohle ist unlöslich in kaltem und heißem Wasser, in organischen Lösemitteln und pflanzlichen Ölen.

Aus dem Stand der Technik sind Veröffentlichungen bekannt, die den Einsatz von Aktivkohle in Reinigungszubereitungen für die Haut und die Haare offenbaren.

EP 1081216 offenbart Seifenzubereitungen, die Aktivkohle enthalten. Diese Zubereitungen sollen ohne weitere Parfümstoffe auskommen, das Haarwachstum fördern und das Haarvolumen vergrößern. Es gibt keine Hinweise auf einen Zusammenhang von verbesserter Reinigungsleistung und dem Einsatz von Aktivkohle.

KR 20080040875 offenbart ebenfalls den Einsatz von Aktivkohle in Shampoo-Zubereitungen. Diese Zubereitungen sollen Dermatitis abmildern und dem Haarausfall vorbeugen.

Das Dokument EP 1982693 offenbart Reinigungszubereitungen, die den Schmutz und abgestorbene Hautpartikel entfernen sollen, u.a. wird der Einsatz von Aktivkohle erwähnt.

In der Offenbarung von EP 1360956 wird eine Reinigungszubereitung offenbart, die Hornzellen, d.h. abgestorbenen Zellen entfernt. Zu diesem Zweck enthält die Zubereitung u.a. Aktivkohle.

Tiefenwirksame aktivkohlehaltige Reinigungsmittel sind ebenso Gegenstand der Dokumente JP 2002167325 und US 6210694.

Des Weiteren gibt es Wettbewerbsprodukte, die Aktivkohle enthalten, wie beispielsweise
- Charcoal Foam Cleanser, Mintel-Datenbank Eintragsnummer 1505578,
- Deep Shower, Mintel-Datenbank Eintragsnummer 891893,
- L'Oréal Face Cleanser, Mintel-Datenbank Eintragsnummer 1776221,
- Palmolive Satstanhos Shampoo, Pelican (Jp), Mintel-Datenbank Eintragsnummer 2665493,
- Clear Unilever (CN), Mintel-Datenbank Eintragsnummer 2261188.

Die Produkte des Standes der Technik sind in der Lage Verschmutzungen zu entfernen und die Hautoberfläche zu reinigen. Derartige Produkte haben jedoch ein weitgehend unattraktives, "unkosmetisches" Aussehen und sprechen den Verbraucher nicht an.

Darüber hinaus ist es wünschenswert, wenn die Aktivkohle, die Form von Partikeln vorliegt, stabil in der Zubereitung verteilt ist und auch weitgehend bleibt.

Die Aufgabe der vorliegenden Erfindung bestand darin, Reinigungsprodukte zur Entfernung von starken Verschmutzungen und zur tiefenwirksamen Reinigung zur Verfügung zu stellen, die darüber hinaus ein kosmetisches Aussehen aufweisen und den Verbraucher ansprechen.

Des Weiteren sollen die Milde und Pflegeleistung der erfindungsgemäßen Zubereitungen wenigstens an die Leistungen von bereits auf dem Markt befindlichen Produkten heranreichen. Überraschend wurde gefunden, dass Reinigungszubereitungen enthaltend Aktivkohle und Perlglanzmittel zu Produkten führen, die ein "kosmetisches" Aussehen aufweisen. Die erfindungsgemäßen Zubereitungen haben ein seidig-graues Aussehen auf, das insbesondere die männlichen Verbraucher anspricht.

Darüber hinaus wurde gefunden, dass der Einsatz bestimmter Strukturanten geeignet ist, eine stabile Verteilung der Partikel zu bewirken.

Die Lösung aller vorgenannten Aufgaben liegt in der Bereitstellung kosmetischer Reinigungszubereitungen für die Haut und/oder die Haare, die neben
- Tensiden, enthaltend anionische und amphotere Tenside, wobei die anionischen Tenside Alkylsulfate und/oder ethoxylierte Alkylsulfate sind und mit einem Gehalt von 2 bis 15 Gew.-%, bezogen auf das Gesamtgewicht der Zubereitung vorliegen,
- Aktivkohle in Form von Aktivkohlepartikeln mit einer Korngröße von 0,5 bis 60 µm, Perlglanzmittel ausgewählt aus der Gruppe Ethylene Glycol Mono-Stearate, Ethylene Glycol Distearate, Polyethylene Glycol Distearate und
- wenigstens ein Acrylatcopolymer und wenigstens einen alkoxylierter Verdicker als Strukturanten, wobei das wenigstens eine Acrylatpolymer ein Acrylates Copolymer ist und wobei der wenigstens eine alkoxylierte Verdicker ausgewählt wird aus der Gruppe PEG-150/Decyl Alcohol/SMDI Copolymer, PEG-150/Stearyl Alcohol/SMDI Copolymer, PEG-200 Glyceryl Tallowate und PEG-200 Hydrogenated Glyceryl Palmate, enthalten.

Als anionische Tenside sind Alkylsulfate und/oder ethoxilierte Alkylsulfate enthalten. Ganz besonders bevorzugt sind derartige Tenside mit einem Alkylrest mit einer Kettenlänge von 10 bis 16 Kohlenstoffatomen. Weiter ganz besonders bevorzugt ist es, wenn als anionisches Tensid Natrium Laurethsulfat eingesetzt wird.

Die anionischen Tenside liegen mit einem Gehalt von 2 bis 15 Gew.-%, bevorzugt 3 bis 12 Gew.-%, insbesondere bevorzugt 4 bis 9 Gew.-%, bezogen auf das Gesamtgewicht der Zubereitung vor.

Es ist erfindungsgemäß vorteilhaft, wenn die Reinigungszubereitungen frei sind von Fettsäuren und Salzen der Fettsäuren, insbesondere frei sind von Seifen. Als "klassische" Seifen werden die Natrium - und Kaliumsalze von Fettsäuren verstanden. Die Fettsäuresalze anderer Metalle nennt man Metallseifen. Die genannten Fettsäuren sind Monocarbonsäuren, die bei der Hydrolyse von Fetten und Ölen frei werden. Sie haben in der Regel wenigstens 8 bis 10, meist jedoch mehr als 14 Kohlenstoffatome. Beispiele sind Laurinsäure, Myristinsäure, Palmitinsäure und andere mehr. "Frei von" im Sinne der vorliegenden Erfindung bedeutet, dass weniger als 0,5 Gew.-%, bevorzugt weniger als 0,1 Gew.-% und ganz besonders bevorzugt weniger als 0,01 Gew.-% an Fettsäuren und/oder Fettsäuresalzen, bezogen auf das Gesamtgewicht der Zubereitung, in den Reinigungszubereitungen vorliegen.

Als amphotere Tenside sind Tenside bevorzugt, die als Grundsubstanz Betain enthalten, wie beispielsweise Alkylbetaine und/oder Alkylamidopropylbetaine. Besonders bevorzugt ist es, wenn das amphotere Tensid ein Cocoamidopropyl Betain ist.

Die amphoteren Tenside liegen mit einem Gehalt von 1 bis 10 Gew.-%, bevorzugt 2 bis 6 Gew.-%, insbesondere bevorzugt 2,5 bis 5 Gew.-%, bezogen auf das Gesamtgewicht der Zubereitung vor.

Möglich ist auch die Einarbeitung von nichtionischen und/oder kationischen Tensiden.

Die Aktivkohle liegt in Form von Partikeln vor. Die Partikel weisen eine Korngröße von D10-D90, entsprechend 0,5-60µm auf. Eine erfindungsgemäß vorteilhafte Aktivkohle ist Cl 77266 Carbon Black oder Charcoal Powder, die beispielsweise unter den Handelsnamen Norit SX Super E 153, Norit A Supra EUR, bei der Firma Brenntag erhältlich sind. Der Gehalt der Aktivkohle in den Reinigungszubereitungen beträgt 0,001 bis 1,0 Gew.-%, bevorzugt 0,01 bis 0,1 Gew.-%, insbesondere bevorzugt 0,01 bis 0,05 Gew.-%, bezogen auf das Gesamtgewicht der Zubereitung.

Die Einarbeitung von Perlglanzmitteln in die erfindungsgemäßen Zubereitungen führt zu perlmuttartig schimmernden Zubereitungen. Produkte mit dieser Optik empfindet der Verbraucher als edel und hochwertig. Die Einarbeitung dieser Pigmente ist jedoch mit Problemen behaftet, weil mit zunehmender Lagerdauer häufig ein Absinken der Pigmente oder eine Ungleich-Verteilung beobachtet wird.

Es gibt Perlglanzmitteln von ganz unterschiedlicher Natur und Zusammensetzung. Die Perlglanzmittel sind aus der Gruppe Ethylene Glycol Mono-Stearate, Ethylene Glycol Distearate, Polyethylene Glycol Distearate ausgewählt, ganz besonders bevorzugt ist das Perlglanzmittel PEG-3 Distearate, das beispielsweise unter dem Handelsnamen Cutina TS von der Firma BASF Personal Care and Nutrition erhältlich ist.

Die Perlglanzmittel liegen mit einem Gehalt von 0,1 bis 3 Gew.-%, bevorzugt 0,2 bis 2 Gew.-%, besonders bevorzugt mit 0,5 bis 1,5 Gew.-%, bezogen auf das Gesamtgewicht der Zubereitung, in den erfindungsgemäßen Zubereitungen vor.

Die erfindungsgemäßen Zubereitungen enthalten Strukturanten. Dies können Substanzen natürlichen oder synthetischen Ursprungs sein. Zum Verständnis der Wirkungsweise von Strukturanten sind verschiedene Eigenschaften wichtig. Zum einen interagieren sie mit Wasser und reduzieren auf diese Weise das Diffusionsbestreben der Wassermoleküle. Darüber hinaus werden Wassermoleküle festgehalten. Dies kann durch Wasserstoffbrückenbindungen geschehen oder Wassermoleküle werden in inter- oder intramolekulare Hohlräume eingelagert. Strukturanten in kosmetischen oder dermatologischen Zubereitungen beeinflussen das Fließverhalten dieser Zubereitungen. Sie werden eingesetzt, um die Viskosität zu erhöhen. Dadurch wird folgenden Phänomen entgegengewirkt: Absetzen von bestimmten Inhaltsstoffen, Phasentrennungen, Kollabieren von Schaum, Kristallisationsvorgänge.

Viele verschiedene Substanzen und Substanzklassen können als Strukturanten wirken. Strukturanten können Polymere sein, die (Meth)Acrylatreste enthalten, beispielsweise Acrylatcolpolymeren in Form von Acrylates/C10-30 Alkyl Acrylates Crosspolymer, Acrylates/Steareth-20 Methacrylate Copolymer, Acrylates Copolymer. Es ist bevorzugt, wenn die diese Acrylatcopolymere nur schwach quervernetzt sind. Erfindungsgemäß eingesetzt werden Polymere vom Typ Acrylates Copolymer, bevorzugt erhältlich unter der Handelsbezeichnung Carbopol Aqua SF-1 Polymer von der Firma Lubrizol Advanced Materials.

Der Gehalt der Acrylatcopolymere beträgt 0,1 bis 3 Gew.-%, bevorzugt 0,4 bis 2,5 Gew.-%, insbesondere bevorzugt 0,6 bis 2,3 Gew.-%, bezogen auf das Gesamtgewicht der Zubereitung.

Ebenso erfindungsgemäß ist der Einsatz von Verdickern, die Alkoxygruppen enthalten. Derartige Verdicker werden erfindungsgemäß gewählt aus PEG-150/Decyl Alcohol/SMDI Copolymer, PEG-150/Stearyl Alcohol/SMDI Copolymer, PEG-200 Glyceryl Tallowate, PEG-200 Hydrogenated Glyceryl Palmate. Erfindungsgemäß besonders bevorzugt ist es, wenn der Verdicker enthaltend Alkoxygruppen PEG-200 Hydrogenated Glyceryl Palmate ist, das beispielsweise erhältlich ist unter der Handelsbezeichnung Rewoderm LI 520-70 von der Firma Evonik Industries.

Der Gehalt der alkoxylierten Verdicker beträgt beträgt 0,1 bis 1,5 Gew.-%, bevorzugt 0,2 bis 1,3 Gew.-%, insbesondere bevorzugt 0,3 bis 1,0 Gew.-%, bezogen auf das Gesamtgewicht der Zubereitung.

Erfindungsgemäß wird eine Kombination von Acrylatcopolymeren und alkoxylierten Verdickern eingesetzt. Es ist vorteilhaft, wenn die Konzentration des Acrylatcopolymers 0,1 bis 3 Gew.-%, bevorzugt 0,6 bis 2,5 Gew.-%, insbesondere bevorzugt 1 bis 2,3 Gew.-%, bezogen auf das Gesamtgewicht der Zubereitung beträgt und der ethoxylierte Verdicker in einer Konzentration von 0,1 bis 1,5 Gew.-%, bevorzugt 0,2 bis 0,9 Gew.-%, insbesondere bevorzugt 0,4 bis 0,6 Gew.-%, bezogen auf das Gesamtgewicht der Zubereitung, vorliegt. Die Begriffe Strukturant und Verdicker sind in der vorliegenden Anmeldung synonym verwendet.

Erfindungsgemäße kosmetische Reinigungszubereitungen gemäß der oben genannten Ausführungen können zur milden, tiefenwirksamen Reinigung von Haut und/oder Haaren in Form eines optisch ansprechenden Shampoos oder Duschgels mit seidiger, metallischer Optik eingesetzt werden.

Die *Tenside,* die in den erfindungsgemäßen Zubereitungen Verwendung finden, umfassen anionische und amphotere sowie ggf. kationische und/oder nichtionische Tenside. Vorteilhaft zu verwendende anionische Tenside sind Acylaminosäuren (und deren Salze), wie
1. Acylglutamate, beispielsweise Natriumacylglutamat, Natrium Cocoylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium-Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat
6. Alaninate

Derivate von Carbonsäuren wie
1. Ester-Carbonsäuren, beispielsweise Calciumstearoyllactylat, Laureth-6-Citrat und Natrium PEG-4-Lauramidcarboxylat,
2. Ether-Carbonsäuren, beispielsweise Natriumlaureth-13-Carboxylat und Natrium PEG-6-Cocamide Carboxylat,

Phosphorsäureester und Salze, wie beispielsweise DEA-Oleth-10-Phosphat und Dilaureth-Phosphat,
Sulfonsäuren und Salze, wie
1. Acyl-isethionate, z.B. Natrium-/ Ammoniumcocoyl-isethionat, Natrium Lauryl Methyl-isethionate,
2. Alkylarylsulfonate,
3. Alkylsulfonate, beispielsweise Natriumcocosmonoglyceridsulfat, Natrium C12-14 Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat, Dinatriumundecylenamido-MEA-Sulfosuccinat und PEG-5 Laurylcitrat Sulfosuccinat.

Erfindungsgemäße Zubereitungen enthalten Schwefelsäureester, umfassend
1. Alkylethersulfat mit unterschiedlichen Ethoxylierungsgraden und deren Gemische, beispielsweise Natrium-, Ammonium-, Magnesium-, MIPA-, TIPA-Laureth-X-sulfat, Natriummyreth-X-sulfat und Natrium C12-13-Pareth-X-sulfat, mit X = 1-5 Ethoxygruppen.
2. Alkylsulfate, beispielsweise Natrium-, Ammonium- und TEA-Laurylsulfat, Natrium- Ammonium- und TEA-Cocosulfat.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Dinatrium Cocoamphodiacetat, Dinatrium Cocoamphomonoacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.
3. Betaine, beispielsweise Coco Betaine, Cocoamidopropyl Betaine,
4. Sultaine, beispielsweise Lauryl Hydroxy Sultaine.

Folgende kationische Tenside können in den erfindungsgemäßen Zubereitungen Verwendung finden:
1. Alkylamine,
2. Alkylimidazole,ethoxylierte Amine
3. Quaternäre Tenside, beispielsweise Cetyl Trimethylammoniumhalogenid.
4. Esterquats, beispielsweise Dicocoylethyl Hydroxyethylmoium Methosulfate und
5. Amidquats, beispielsweise Palmitamidopropyltrimonium Chlorid.

Quaternäre Tenside enthalten mindestens ein N-Atom, das mit 4 Alkyl- und/oder Arylgruppen kovalent verbunden ist. Dies führt, unabhängig vom pH Wert, zu einer positiven Ladung. Vorteilhafte quaternäre Tenside sind Alkylbetain, Alkylamidopropylbetain und Alkyl-amidopropylhydroxysulfain. Kationische Tenside können ferner bevorzugt im Sinne der vorliegenden Erfindung gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder-bromid, Alkyldimethylhydroxyethylammoniumchloride oder-bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Folgende nichtionische Tenside können in den erfindungsgemäßen Zubereitungen Verwendung finden:
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, Laureth-X mit X = 2 bis 10, wobei X Ethoxygruppe bedeutet, ethoxylierte/ propoxylierte Ester, ethoxylierte/ propoxylierte Glycerinester, PEG-200 Hydrogenated Glyceryl Palmat, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Um den erfindungsgemäßen Zubereitungen rückfettende Eigenschaften zu verleihen, können beispielsweise *Öle* in die erfindungsgemäßen Zubereitungen eingearbeitet werden.

Die Öle können ausgewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat,lsopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* erhältlich ist.

Es ist ferner bevorzugt, die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C12-13-Alkyllactat, Di-C12-13-Alkyltartrat, Triisostearin, Dipentaerythrityl Hexacaprylat/Hexacaprat, Propylenglykolmonoisostearat, Tricaprylin, Dimethylisosorbid zu wählen. Es ist insbesondere vorteilhaft, wenn die Ölphase der erfindungsgemäßen Formulierungen einen Gehalt an C12-15-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* erhältliche), Hexadecylbenzoat und Butyloctylbenzoat und Gemische davon (*Hallstar AB*) und/oder Diethylhexylnaphthalat (*Hallbrite TQ oder Corapan TQ von H&R*)*.*

Auch beliebige Abmischungen solcher Öl- und Wachskomponenten sind vorteilhaft im Sinne der vorliegenden Erfindung einzusetzen.

Erfindungsgemäß sind auch Substanzen, die üblicherweise eingesetzt werden, um den pH-Wert der erfindungsgemäßen Zubereitungen einzustellen und stabil zu halten. Vorteilhaft können diese Substanzen ausgewählt werden aus der Gruppe Zitronensäure und Milchsäure.

Die erfindungsgemäßen Reinigungszubereitungen können kosmetische Hilfsstoffe enthalten, wie sie üblicherweise in solchen Zubereitungen verwendet werden, z.B. Konservierungsmittel, Bakterizide, Parfüme, Farbstoffe, Pigmente, die eine färbende Wirkung haben, Emulgatoren, weichmachende, anfeuchtende und/oder feuchthaltende Substanzen oder andere übliche Bestandteile einer kosmetischen oder dermatologischen Formulierung wie Alkohole, Polyole, Schaumstabilisatoren, organische Lösemittel oder Silikonderivate.

Die erfindungsgemäßen Zubereitungen liegen in den bekannten galenischen Formen vor, wie sie für Reinigungs- und Shampoozubereitungen üblich sind. Bevorzugt sind allerdings flüssige, fließfähige oder gelartige Ausführungsformen.

Die Bestimmung der *Milde* wird mit Hilfe eines RBC-Assays durchgeführt.

Der Standard-RBC-Assay (10 Minuten Inkubation) dient zur Abschätzung von in vivo Augenschleimhautreizpotentialen von Tensiden und tensidhaltigen Produkten.

### 1. Hämolvse

Ein definiertes Aliquot isolierter Kalbserythrozyten wird mit einer Reihe steigender Konzentrationen des zu untersuchenden WAS-Prüfmusters (Stammlösung mit Formulierungen 1:100 w/v bzw. für Rohstoffe 0.1 % Aktivgehalt in PBS) für 10 Minuten unter Schütteln bei Raumtemperatur (RT) inkubiert. Nach Zentrifugation werden die gewonnenen Überstände photometrisch auf ihren Gehalt an freigesetztem Hämoglobin (HbO₂) bei 530 nm analysiert. Daraus wird der relative Hämolysegrad berechnet und die Konzentrations-Response-Kurve mit dem H50-Wert [µl/ml] als Kenngröße bestimmt. Dieser gibt die Konzentration des Prüfmusters an, bei der 50% des Hämoglobins freigesetzt werden.

### 2. HbO₂-Denaturierung

Ein definiertes Aliquot isolierter Kalbserythrozyten wird mit einer fixen Konzentration des Prüfmusters (1% w/v bzw. 0.1% Aktivgehalt) für 10 Minuten unter Schütteln bei RT inkubiert und dann zentrifugiert. Die Änderung der spektralen Absorption bei 575 nm und 540 nm wird im Vergleich zum nativen HbO₂ gemessen. Aus dem Verhältnis der Absorptionswerte zueinander wird der Denaturierungsindex DI [%] berechnet. Als 100%-Standard dient Na-Laurylsulfat (0.1% Aktivgehalt).

### 3. L/D-Quotient

Der Quotient stellt das Verhältnis der Kenngrößen von Hämolyse (H50) und Denaturierung (Dl) dar und wird zur Charakterisierung und Klassifizierung der untersuchten Prüfmuster verwendet.

Die oben beschriebene Methode zur Bestimmung der Milde wurde von Pape et al. In Molecular Toxicology, 1, 525-536, 1987 veröffentlicht.

Ausgewählte erfindungsgemäße Zubereitungen wurden auf verschiedene Weise untersucht, um zu belegen, dass die vorgenannten Aufgaben gelöst werden.

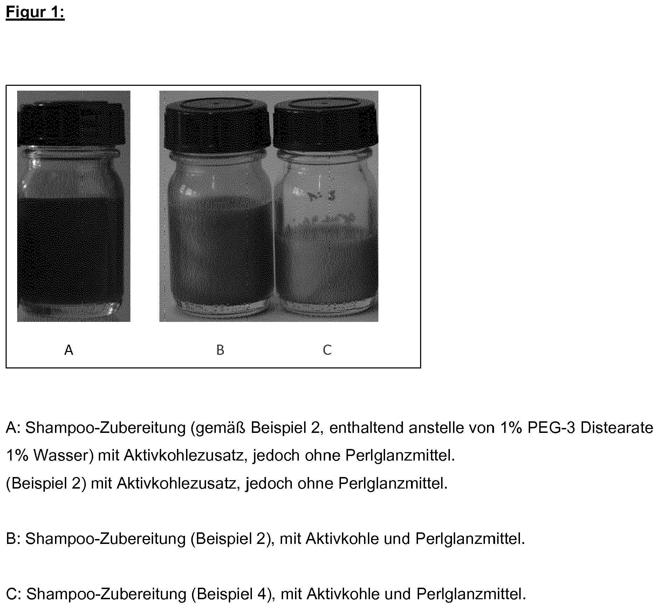

Um die Verbesserung der erfindungsgemäßen Zubereitungen in Bezug auf das Aussehen zu belegen, wurde die Zusammensetzung Nr. 2 der Shampoo-Beispielrezepturen mit und ohne Perlglanzmittel hergestellt; anschließend wurden die Ansätze fotografiert. Zusätzlich wurde die Zusammensetzung Nr. 4 hergestellt und ebenfalls fotografiert; siehe dazu Figur 1. Es wird deutlich, dass die Perlglanzmittel das Aussehen der Zusammensetzungen nachhaltig verbessern. Die erfindungsgemäßen Zubereitungen weisen eine graue Farbe auf und einen ansprechenden seidigen oder auch metallischen Glanz.

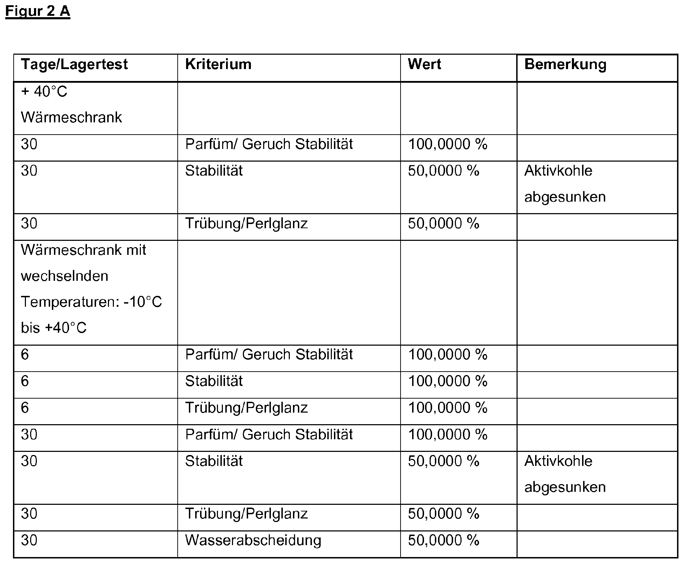

Die Lagerstabilität einer ausgewählten nicht erfindungsgemäßen Shampoozubereitung, Beispiel 3 der Shampoozubereitungen wurde untersucht. Statt der erfindungsgemäßen Verdicker Acrylates Copolymer und PEG-200 Hydrogenated Glyceryl Palmate wurde ein nicht erfindungsgemäßer Verdicker, nämlich Acrylates/ C 10-30 Alkyl Acrylate Crosspolymer (Carbopol ETD2020, Lubrizol Advanced Materials) eingearbeitet. Nach 30 Tagen Lagerung in einem Brutschrank bei 40°C oder Lagerung in einem Brutschrank mit von -10°C bis +40°C regelmäßig wechselnden Temperaturen war die Aktivkohle abgesunken, siehe dazu Figur 2A.

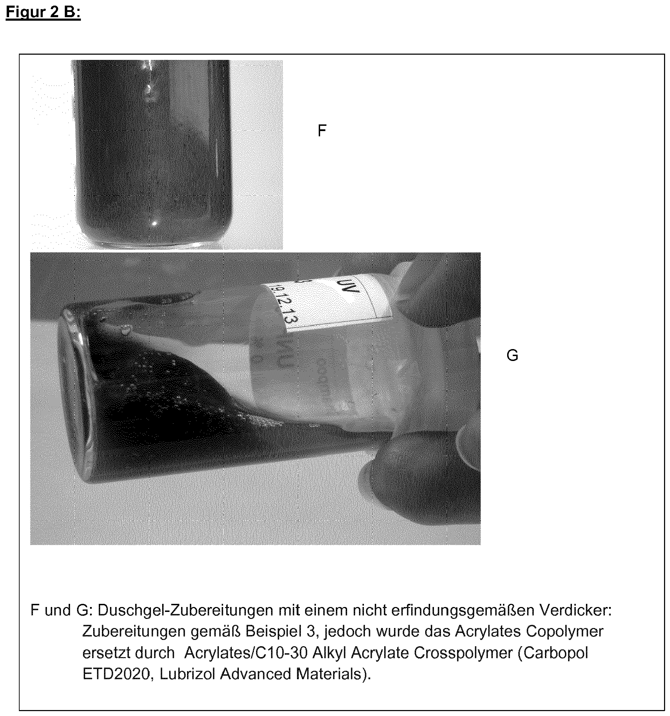

In Figur 2 B werden weitere Ergebnisse zur Lagerstabilität dargestellt. Eine Duschgelzubereitungen gemäß Beipielrezeptur 3 wurde hergestellt, wobei ein nicht erfindungsgemäßer Verdicker, nämlich Acrylates/ C10-30 Alkyl Acrylate Crosspolymer (Carbopol ETD2020, Lubrizol Advanced Materials) statt eines Acrylaten Copolymers eingearbeitet wurde. Es wird deutlich, dass nach 30 Tagen die Aktivkohle-Partikel auf den Boden abgesunken sind, siehe Probe F in Figur 2 B. Ebenso kommt es bei Einarbeitung eines nicht erfindungsgemäßen Verdickers nach 30 Tagen zur Phasentrennung, siehe Probe G in Figur 2 B. Es wird deutlich, dass nur die Verwendung eines erfindungsgemäßen Verdickers zu Zubereitungen führt, die stabil sind und die eine homogene Verteilung der Aktivkohle-Partikel bewirken.

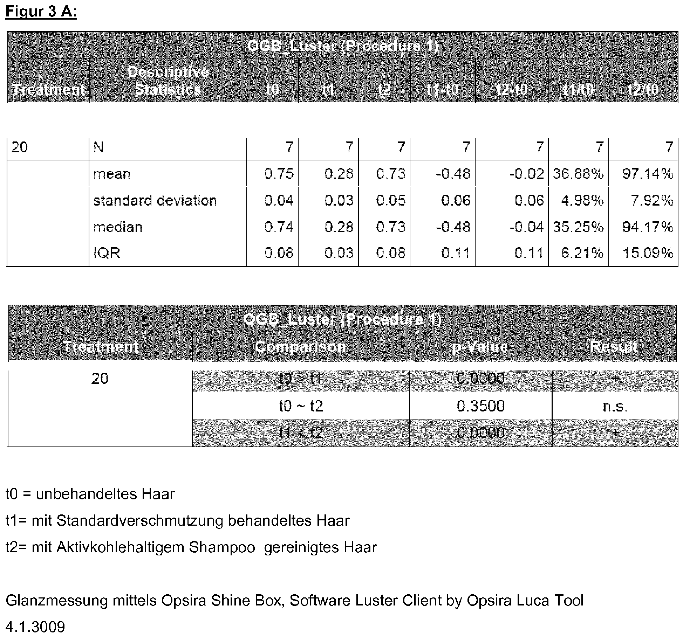

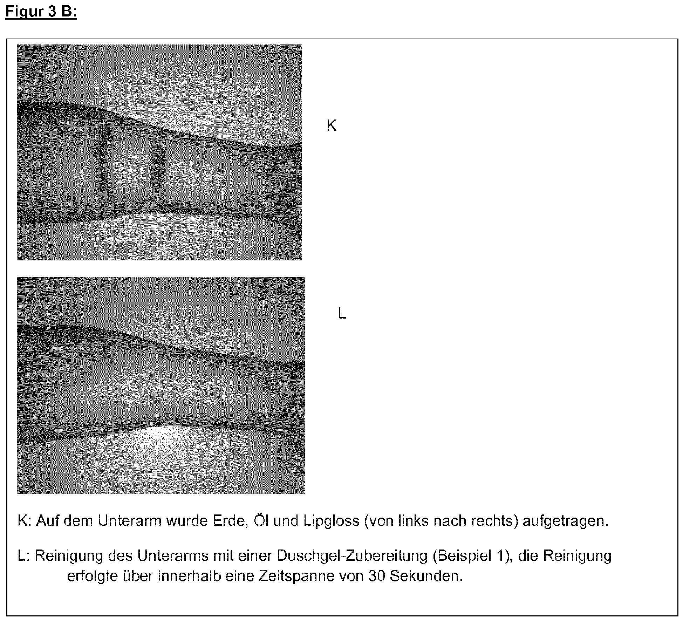

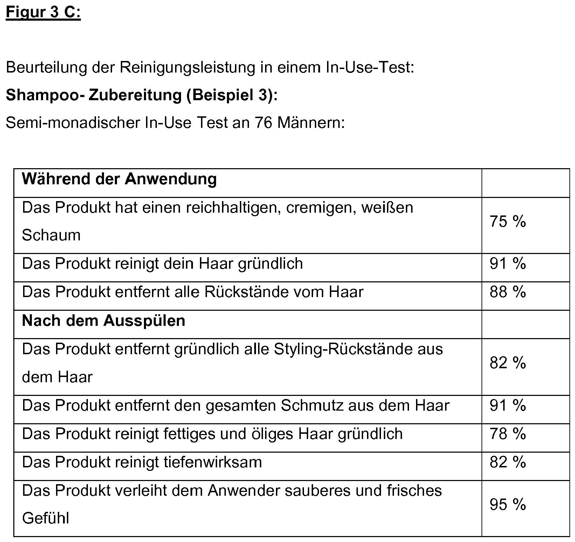

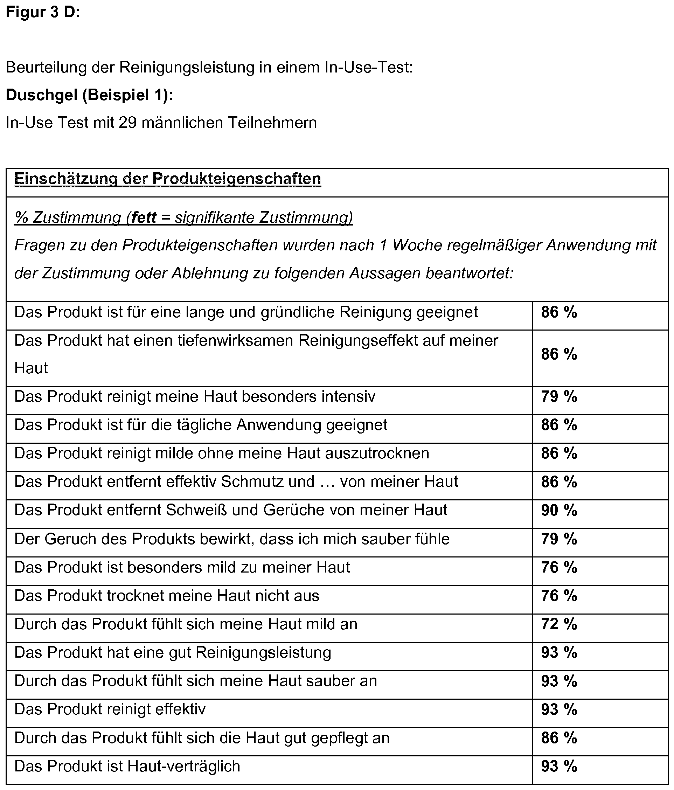

In den Abbildungen und Tabellen der Figur 3 sind Ergebnisse dargestellt, die die Reinigungsleistung ausgewählter erfindungsgemäßer Zubereitungen belegen.

In Figur 3 A wird die Effizienz in Bezug auf die Reinigungsleistung einer erfindungsgemäßen Haarzubereitung gezeigt. Dazu wird eine Haartresse von ungeschädigtem Humanhaar (Art. 826502, Kerling) einer Standardverschmutzung unterzogen. Die Erzeugung der Standardverschmutzung besteht aus 10-minütigem Eintauchen der trockenen Haarsträhne in 0,1%ige Grundseifenlösung mit anschließendem 10 minütigen Eintauchen in Wasser mit 42 dH °. Dabei besteht die Grundseifenlösung aus Sodium Tallowate, Sodium Cocoate, Aqua, Glycerin, Sodium Chloride, Tetrasodium Etidronate / Grundseife 80/20, Hirtler). Nach einer Trocknungsphase wird das Haar mit einer erfindungsgemäßen Zubereitung gewaschen. Das Haar wird vor der Verschmutzung, nach der Verschmutzung und nach dem Waschen einer Glanzmessung unterzogen mittels einer Opsira Shine Box, Software Luster Client by Opsira Luca Tool 4.1.3009. Die Ergebnisse zeigen erwartungsgemäß signifikante Unterschiede im Vergleich von unverschmutzem zu verschmutztem Haar und verschmutztem zu gewaschenem Haar. Der Unterschied von unverschmutztem zu gewaschenem Haar ist jedoch nicht signifikant, Werte von 0,75 und 0,73 werden erreicht. Dies bedeutet, dass der Glanz des gewaschenen Haares in derselben Größenordnung liegt, wie derjenige des unverschmutzten Haares. Mit der erfindungsgemäßen Zubereitung wurde also der Schmutz vollständig entfernt.

Die Abbildung der Figur 3 B zeigt, dass Verschmutzungen bestehend aus Erde, Öl oder Lipgloss mit einem üblichen Reinigungsschritt durch eine ausgewählte erfindungsgemäße Zubereitung wieder vollständig von der Haut entfernt werden können.

In Figur 3 C werden die Ergebnisse eines In-Use Tests dargestellt. 76 Männer verwendeten das Haarshampoo Beispiel 3 für 7 Tage, mindestens jedoch 3-mal. Anschließend wurden die Bewertungen mit Hilfe eines Fragebogens ermittelt. Die Anwender bewerteten das getestete Produkt u.a. in Bezug auf den Schaum, die Reinigungsleistung und die Entfernung von Rückständen im Haar. Die Bewertungen hinsichtlich dieser Eigenschaften fielen überdurchschnittlich gut aus.

In Figur 3 D sind die Ergebnisse eines In-Use Tests für eine ausgewählte Duschzubereitung dargestellt. 26 Männer im Alter von 22-64 Jahren verwendeten das Duschgel Beispiel 1 für 14 Tage jeweils 1 Mal täglich. Die Bewertungen wurden mit Hilfe eines Fragebogens ermittelt. Die Ergebnisse zeigen, dass das Produkt eine tiefenwirksame Reinigungsleistung aufweist, effektiv Schmutz und andere Rückstände von der Haut entfernt, mild zur Haut ist und zur Pflege der Haut beiträgt.

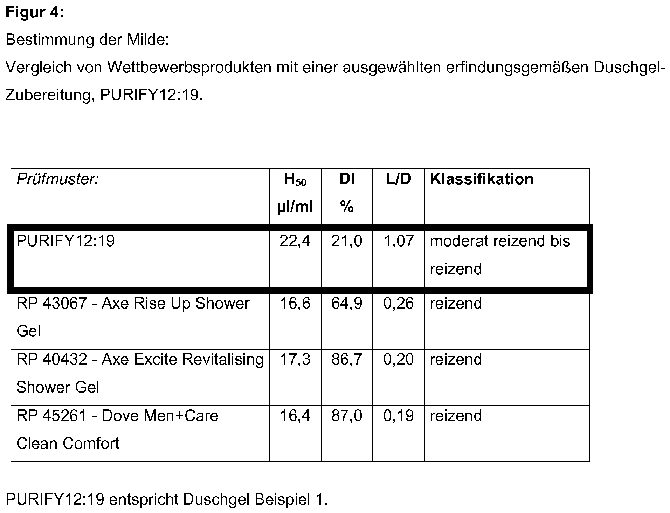

Die Ergebnisse zur Bestimmung der Milde mit einem Hämolysetest unter Verwendung von Kalbserythrozyten sind in Figur 4 dargestellt. Eine ausgewählte erfindungsgemäße Zubereitung, PURIFY12:19, zusammen mit drei verschiedenen Produkten, die käuflich zu erwerben sind, wurde analysiert. Die Ergebnisse zeigen, dass die erfindungsgemäße Zubereitung in diesem Test als moderat reizend bis reizend eingestuft wird. Der ermittelte Wert ist jedoch mit etwa einen Faktor 5 besser ist als diejenigen Werte der käuflich zu erwerbenden Produkte.

### Beispiele:

### Beispiele für Shampoo-Zubereitungen (die Beispiele 1, 2 und 4-6 sind nicht erfindungsgemäß) :

| | | **w/w % (Aktivgehalt** | | | | | |
|---|---|---|---|---|---|---|---|
| | INCI | 1 | 2 | 3 | 4 | 5 | 6 |
| **1** | Sodium Laureth Sulfate | 7 | 4,6 | 7 | 8 | 7 | 8,5 |
| **2** | Cocamidopropyl Betaine | 3,4 | 4,5 | 3,4 | 3,3 | 2,7 | 4,2 |
| **3** | Disodium Cocoyl Glutamate | - | 0,5 | - | - | - | - |
| **4** | Acrylates Copolymer | 1,8 | 2,25 | 1,8 | - | - | - |
| **5** | PEG-3 Distearate | 1 | 1 | 1 | 0,8 | 1 | 1,5 |
| **6** | PEG-7 Glyceryl Cocoate | - | 1,75 | - | - | - | - |
| **7** | PEG-40 Hydrogenated Castor Oil | 0,5 | 0,5 | 0,5 | 0,5 | 0,5 | 0,5 |
| **8** | Cl 77266 | 0,05 | 0,05 | 0,015 | 0,001 | 0,010 | 0,005 |
| **9** | PEG-200 Hydrogenated Glyceryl Palmate | - | - | 0,2 | 0,5 | 0,3 | 0,5 |
| **1 0** | Citric Acid | 0,2 | - | 0,8 | 0,5 | 0,5 | 0,4 |
| **11** | Sodium Chloride | - | - | - | 0,2 | 0,5 | 0,5 |
| | Parfum, Konservierung, Extrakte, Farbstoffe | q.s. | q.s. | q.s. | q.s. | q.s. | q.s. |
| | Aqua | ad. 100 | ad. 100 | ad. 100 | ad. 100 | ad. 100 | ad. 100 |

### Handelsnamen der verwendeten Rohstoffe:

| | |
|---|---|
| 1 | Texapon N70, BASF Personal Care and Nutrition / Galaxy LES 70, Galaxy Surfactants / Hansanol NS 242 conc., Wibargo |
| 2 | Tego-Betain F 50, Evonik Industries |
| 3 | Protelan AGL 95/C, Zschimmer&Schwarz / Hostapon CCg, Clariant |
| 4 | Carbopol Aqua SF-1 Polymer, Lubrizol Advanced Materials |
| 5 | Cutina TS, BASF Personal Care and Nutrition |
| 6 | Cetiol HE, BASF Personal Care and Nutrition / Tegosoft GC, Evonik Industries / Glycerox HE B, Croda |
| 7 | Eumulgin CO 40, BASF Personal Care and Nutrition / Sympatens TRH/400, Kolb |
| 8 | Norit SX Super E 153, Brenntag |
| 9 | Rewoderm LI 520-70, Evonik Industries |
| 10 | CITRIC ACID MONOHYDRATE FINE GRANULAR 04 3277 6, Brenntag |
| 11 | Suprasel fine, Akzo Nobel / Jurasel Speisesalz, Schweizer Rheinsalinen |

### Beispiele für Duschgele (das Beispiel 5 ist nicht erfindungsgemäß):

| | | w/w % (Aktivgehalt) | | | | |
|---|---|---|---|---|---|---|
| | **Inci** | **1** | **2** | **3** | **4** | **5** |
| 1 | Sodium Laureth Sulfate | 7 | 6 | 8 | 9 | 6 |
| 2 | Cocamidopropyl Betaine | 4,5 | 4 | 5 | 3 | 4 |
| 3 | Acrylates Copolymer | 1 | 0,6 | 1,5 | 1,5 | |
| 4 | PEG-7 Glyceryl Cocoate | 1 | 0,5 | 1,4 | 1 | 0,5 |
| 5 | PEG-3 Distearate | 0,5 | - | 0,2 | 0,9 | 0,5 |
| 6 | Glycol Distearate | - | 0,8 | - | 0,6 | |
| 7 | PEG-200 Hydrogenated Glyceryl Palmate | 0,5 | 0,2 | 1,3 | 0,9 | 1,3 |
| 8 | PEG-40 Hydrogenated Castor Oil | 0,5 | 0,2 | 0,2 | 1 | 0,2 |
| 9 | Cl 77266 (Carbon Black) oder Charcoal Powder | 0,02 | 0,05 | 0,01 | 0,1 | 0,05 |
| 1 0 | Acrylates/C10-30 Alkyl Acrylate Crosspolymer | | | | | 0,5 |
| | Parfüm, Konservierung, Extrakte | Qs | qs | qs | qs | qs |
| | Wasser | ad 100 | ad 100 | ad 100 | ad 100 | ad 100 |

### Handelsnamen der verwendeten Rohstoffe:

| | |
|---|---|
| 1 | Texapon N70, BASF Personal Care and Nutrition / Galaxy LES 70, Galaxy Surfactants / Hansanol NS 242 conc., Wibargo |
| 2 | Tego-Betain F 50, Evonik Industries |
| 3 | ACEcare30KC, KCl / Carbopol Aqua SF-1 Polymer, Lubrizol Advanced Materials |
| 4 | Cetiol HE, BASF Personal Care and Nutrition / Tegosoft GC, Evonik Industries / Glycerox HE B, Croda |
| 5 | Cutina TS, BASF Personal Care and Nutrition |
| 6 | Euperlan PK 3000 OK, BASF Personal Care and Nutrition |
| 7 | Rewoderm LI 520-70, Evonik Industries |
| 8 | Eumulgin CO 40, BASF Personal Care and Nutrition / Sympatens TRH/400, Kolb |
| 9 | Norit SX Super E 153, Brenntag (Cabot Norit) |
| 10 | Carbopol ETD 2020, Lubrizol Advanced Material |

## Patentansprüche

1. Kosmetische Reinigungszubereitungen für die Haut und/oder die Haare enthaltend
• Tenside, enthaltend anionische und amphotere Tenside,
wobei die anionischen Tenside Alkylsulfate und/oder ethoxylierte Alkylsulfate sind und mit einem Gehalt von 2 bis 15 Gew.-%, bezogen auf das Gesamtgewicht der Zubereitung vorliegen,
• Aktivkohle in Form von Aktivkohlepartikeln mit einer Korngröße von 0,5 bis 60 µm,
• Perlglanzmittel, auswählt aus der Gruppe Ethylene Glycol Mono-Stearate, Ethylene Glycol Distearate und Polyethylene Glycol Distearate,
• wenigstens ein Acrylatcopolymer und wenigstens ein alkoxylierter Verdicker als Strukturanten,
wobei das wenigstens eine Acrylatpolymer ein Acrylates Copolymer ist und wobei der wenigstens eine alkoxylierte Verdicker ausgewählt wird aus der Gruppe PEG-150/Decyl Alcohol/SMDI Copolymer, PEG-150/Stearyl Alcohol/SMDI Copolymer, PEG-200 Glyceryl Tallowate und PEG-200 Hydrogenated Glyceryl Palmate.

2. Kosmetische Reinigungszubereitungen nach Anspruch 1, **dadurch gekennzeichnet, dass** die anionischen Tenside Alkylsulfate und/oder ethoxilierte Alkylsulfate mit einem Alkylrest mit einer Kettenlänge von 10 bis 16 Kohlenstoffatomen, insbesondere bevorzugt Natrium Laurethsulfat sind.

3. Kosmetische Reinigungszubereitungen nach wenigstens einem der vorstehenden Ansprüche, **dadurch gekennzeichnet, dass** die anionischen Tenside mit einem Gehalt von 3 bis 12 Gew.-%, bevorzugt 4 bis 9 Gew.-%, bezogen auf das Gesamtgewicht der Zubereitung vorliegen.

4. Kosmetische Reinigungszubereitungen nach wenigstens einem der vorstehenden Ansprüche, **dadurch gekennzeichnet, dass** die Reinigungszubereitungen frei sind von Fettsäuren und Salzen der Fettsäuren, insbesondere frei von Seifen.

5. Kosmetische Reinigungszubereitungen nach wenigstens einem der vorstehenden Ansprüche, **dadurch gekennzeichnet, dass** die amphotere Tenside Alkylbetaine und/oder Alkylamidopropylbetaine, bevorzugt Cocoamidopropyl Betaine sind.

6. Kosmetische Reinigungszubereitungen nach wenigstens einem der vorstehenden Ansprüche, **dadurch gekennzeichnet, dass** die amphoteren Tenside mit einem Gehalt von 1 bis 10 Gew.-%, bevorzugt 2 bis 6 Gew.-%, insbesondere bevorzugt 2,5 bis 5 Gew.-%, bezogen auf das Gesamtgewicht der Zubereitung vorliegen.

7. Kosmetische Reinigungszubereitungen nach wenigstens einem der vorstehenden Ansprüche, **dadurch gekennzeichnet, dass** die Aktivkohle Cl 77266 Carbon Black oder Charcoal Powder ist.

8. Kosmetische Reinigungszubereitungen nach wenigstens einem der vorstehenden Ansprüche, **dadurch gekennzeichnet, dass** der Gehalt der Aktivkohle in den Reinigungszubereitungen 0,001 bis 1 Gew.-%, bevorzugt 0,01 bis 0,1 Gew.-%, insbesondere bevorzugt 0,01 bis 0,05 Gew.-%, bezogen auf das Gesamtgewicht der Zubereitung, beträgt.

9. Kosmetische Reinigungszubereitungen nach wenigstens einem der vorstehenden Ansprüche, **dadurch gekennzeichnet, dass** das Perlglanzmittel PEG-3 Distearate ist.

10. Kosmetische Reinigungszubereitungen nach wenigstens einem der vorstehenden Ansprüche, **dadurch gekennzeichnet, dass** die Perlglanzmittel mit einem Gehalt von 0,1 bis 3 Gew.-%, bevorzugt 0,2 bis 2 Gew.-%, besonders bevorzugt mit 0,5 bis 1,5 Gew.-%, bezogen auf das Gesamtgewicht der Zubereitung, vorliegen.

11. Kosmetische Reinigungszubereitungen nach wenigstens einem der vorstehenden Ansprüche, **dadurch gekennzeichnet, dass** der Verdicker enthaltend Alkoxygruppen PEG-200 Hydrogenated Glyceryl Palmate ist.

12. Kosmetische Reinigungszubereitungen nach wenigstens einem der vorstehenden Ansprüche, **dadurch gekennzeichnet, dass** das wenigstens eine Acrylatcopolymere in einer Konzentration von 0,1 bis 3 Gew.-%, bevorzugt 0,6 bis 2,5 Gew.-%, insbesondere bevorzugt 1 bis 2,3 Gew.-%, bezogen auf das Gesamtgewicht der Zubereitung und der wenigstens eine ethoxylierte Verdicker in einer Konzentration von 0,1 bis 1,5 Gew.-%, bevorzugt 0,2 bis 0,9 Gew.-%, insbesondere bevorzugt 0,4 bis 0,6 Gew.-%, bezogen auf das Gesamtgewicht der Zubereitung, vorliegt.

## Claims

1. Cosmetic cleansing preparations for skin and/or hair comprising
• surfactants, comprising anionic and amphoteric surfactants,
wherein the anionic surfactants are alkyl sulfates and/or ethoxylated alkyl sulfates and are present at a content of 2 to 15% by weight, based on the total weight of the preparation,
• activated carbon in the form of activated carbon particles having a particle size of 0.5 to 60 µm,
• pearlizing agent selected from the group comprising ethylene glycol monostearate, ethylene glycol distearate and polyethylene glycol distearate,
• at least one acrylate copolymer and at least one alkoxylated thickener as structurants,
wherein the at least one acrylate polymer is an acrylate copolymer and wherein the at least one alkoxylated thickener is selected from the group comprising PEG-150/decyl alcohol/SMDI copolymer, PEG-150/stearyl alcohol/SMDI copolymer, PEG-200 glyceryl tallowate and PEG-200 hydrogenated glyceryl palmate.

2. Cosmetic cleansing preparations according to Claim 1, **characterized in that** the anionic surfactants are alkyl sulfates and/or ethoxylated alkyl sulfates with an alkyl radical having a chain length of 10 to 16 carbon atoms, particularly preferably sodium laureth sulfate.

3. Cosmetic cleansing preparations according to at least one of the preceding claims, **characterized in that** the anionic surfactants are present at a content of 3 to 12% by weight, preferably 4 to 9% by weight, based on the total weight of the preparation.

4. Cosmetic cleansing preparations according to at least one of the preceding claims, **characterized in that** the cleansing preparations are free of fatty acids and salts of fatty acids, especially free of soaps.

5. Cosmetic cleansing preparations according to at least one of the preceding claims, **characterized in that** the amphoteric surfactants are alkyl betaines and/or alkylamidopropyl betaines, preferably cocoamidopropyl betaines.

6. Cosmetic cleansing preparations according to at least one of the preceding claims, **characterized in that** the amphoteric surfactants are present at a content of 1 to 10% by weight, preferably 2 to 6% by weight, particularly preferably 2.5 to 5% by weight, based on the total weight of the preparation.

7. Cosmetic cleansing preparations according to at least one of the preceding claims, **characterized in that** the activated carbon is Cl 77266 carbon black or charcoal powder.

8. Cosmetic cleansing preparations according to at least one of the preceding claims, **characterized in that** the content of activated carbon in the cleansing preparations is 0.001 to 1% by weight, preferably 0.01 to 0.1% by weight, particularly preferably 0.01 to 0.05% by weight, based on the total weight of the preparation.

9. Cosmetic cleansing preparations according to at least one of the preceding claims, **characterized in that** the pearlizing agent is PEG-3 distearate.

10. Cosmetic cleansing preparations according to at least one of the preceding claims, **characterized in that** the pearlizing agent is present at a content of 0.1 to 3% by weight, preferably 0.2 to 2% by weight, particularly preferably 0.5 to 1.5% by weight, based on the total weight of the preparation.

11. Cosmetic cleansing preparations according to at least one of the preceding claims, **characterized in that** thickener comprising alkoxy groups is PEG-200 hydrogenated glyceryl palmate.

12. Cosmetic cleansing preparations according to at least one of the preceding claims, **characterized in that** the at least one acrylate copolymer is present at a concentration of 0.1 to 3% by weight, preferably 0.6 to 2.5% by weight, particularly preferably 1 to 2.3% by weight, based on the total weight of the preparation and the at least one ethoxylated thickener is present at a concentration of 0.1 to 1.5% by weight, preferably 0.2 to 0.9% by weight, particularly preferably 0.4 to 0.6% by weight, based on the total weight of the preparation.

## Revendications

1. Préparations de nettoyage cosmétiques pour la peau et/ou les cheveux contenant
• des tensioactifs, contenant des tensioactifs anioniques et amphotères, les tensioactifs anioniques étant des alkylsulfates et/ou des alkylsulfates éthoxylés et étant présents en une teneur de 2 à 15 % en poids, par rapport au poids total de la préparation,
• du charbon actif sous forme de particules de charbon actif dotées d'une grosseur de grain de 0,5 à 60 µm,
• un agent nacrant, choisi dans le groupe du monostéarate d'éthylèneglycol, du distéarate d'éthylèneglycol et du distéarate de polyéthylène glycol,
• au moins un copolymère d'acrylate et au moins un épaississant alcoxylé en tant qu'agents structurants,
l'au moins un polymère d'acrylate étant un copolymère d'acrylate et l'au moins un épaississant alcoxylé étant choisi dans le groupe d'un copolymère PEG-150/alcool décylique/SMDI, un copolymère PEG-150/alcool stéarique/SMDI, le PEG-200-tallowate de glycéryle et le PEG-200-palmate de glycéryle hydrogéné.

2. Préparations de nettoyage cosmétiques selon la revendication 1, **caractérisées en ce que** les tensioactifs anioniques sont des alkylsulfates et/ou des alkylsulfates éthoxylés comportant un radical alkyle doté d'une longueur de chaîne de 10 à 16 atomes de carbone, en particulier préférablement sont le laurethsulfate de sodium.

3. Préparations de nettoyage cosmétiques selon au moins l'une des revendications précédentes, **caractérisées en ce que** les tensioactifs anioniques sont présents en une teneur de 3 à 12 % en poids, préférablement de 4 à 9 % en poids, par rapport au poids total de la préparation.

4. Préparations de nettoyage cosmétiques selon au moins l'une des revendications précédentes, **caractérisées en ce que** les préparations de nettoyage sont exemptes d'acides gras et de sels des acides gras, en particulier exemptes de savons.

5. Préparations de nettoyage cosmétiques selon au moins l'une des revendications précédentes, **caractérisées en ce que** les tensioactifs amphotères sont des alkylbétaïnes et/ou des alkylamidopropylbétaïnes, préférablement sont des cocoamidopropylbétaïnes.

6. Préparations de nettoyage cosmétiques selon au moins l'une des revendications précédentes, **caractérisées en ce que** les tensioactifs amphotères sont présents en une teneur de 1 à 10 % en poids, préférablement de 2 à 6 % en poids, en particulier préférablement de 2,5 à 5 % en poids, par rapport au poids total de la préparation.

7. Préparations de nettoyage cosmétiques selon au moins l'une des revendications précédentes, **caractérisées en ce que** le charbon actif est le noir de charbon Cl 77266 ou la poudre de charbon.

8. Préparations de nettoyage cosmétiques selon au moins l'une des revendications précédentes, **caractérisées en ce que** la teneur en charbon actif dans les préparations de nettoyage est de 0,001 à 1 % en poids, préférablement de 0,01 à 0,1 % en poids, en particulier préférablement de 0,01 à 0,05 % en poids, par rapport au poids total de la préparation.

9. Préparations de nettoyage cosmétiques selon au moins l'une des revendications précédentes, **caractérisées en ce que** l'agent nacrant est le distéarate de PEG-3.

10. Préparations de nettoyage cosmétiques selon au moins l'une des revendications précédentes, **caractérisées en ce que** les agents nacrants sont présents en une teneur de 0,1 à 3 % en poids, préférablement de 0,2 à 2 % en poids, particulièrement préférablement avec 0,5 à 1,5 % en poids, par rapport au poids total de la préparation.

11. Préparations de nettoyage cosmétiques selon au moins l'une des revendications précédentes, **caractérisées en ce que** l'épaississant contenant des groupes alcoxy est le PEG-200-palmate de glycéryle hydrogéné.

12. Préparations de nettoyage cosmétiques selon au moins l'une des revendications précédentes, **caractérisées en ce que** l'au moins un copolymère d'acrylate est présent en une concentration de 0,1 à 3 % en poids, préférablement de 0,6 à 2,5 % en poids, en particulier préférablement de 1 à 2,3 % en poids, par rapport au poids total de la préparation et l'au moins un épaississant éthoxylé est présent en une concentration de 0,1 à 1,5 % en poids, préférablement de 0,2 à 0,9 % en poids, en particulier préférablement de 0,4 à 0,6 % en poids, par rapport au poids total de la préparation.
